# EUROPÄISCHE PATENTANMELDUNG

(11) **EP 0 661 021 A2**
(43) Veröffentlichungstag der Anmeldung: **05.07.1995**
(21) Anmeldenummer: 94115710.9
(22) Anmeldetag: 06.10.1994
(51) Int. Cl.: A61B 10/00, A61B 5/00

(54) **Probenabnahmebesteck zur Gewinnung von Tränenflüssigkeit**

(30) Priorität: 21.10.1993 DE 4335961
(71) Anmelder: BEHRINGWERKE Aktiengesellschaft, 35001 Marburg (DE)
(72) Erfinder: Römisch, Jürgen, Dr., D-35041 Marburg (DE); Schüler, Eckhard, Dr., D-35041 Marburg (DE); Habenstein, Klaus, Dr., D-35083 Wetter (DE); Lindner, Jürgen, Dr., D-35041 Marburg (DE)

(57) **Zusammenfassung**

Die Erfindung betrifft ein Probenabnahmebesteck zur schnellen und schonenden Gewinnung eines definierten Volumens Tränenflüssigkeit zur reproduzierbaren Bestimmung von Komponenten der Tränenflüssigkeit.

## Beschreibung

Die Erfindung betrifft ein Probenabnahmebesteck zur schnellen und schonenden Gewinnung eines definierten Volumens Tränenflüssigkeit zur reproduzierbaren Bestimmung von Komponenten der Tränenflüssigkeit.

Im Verlauf der Pathogenese von ophthalmologischen Erkrankungen können sich Konzentrationen verschiedener Komponenten ändern. Darunter befinden sich Enzyme, z.B. Proteasen, deren Aktivitätsab- oder -zunahmen Hinweise auf Irritationen oder Schädigungen, vor allem des vorderen Augenabschnittes geben können. Sowohl zur Diagnose einer Erkrankung als auch zum "Monitoring" eines Therapieverlaufes sind solche Quantifizierungen bestimmter Parameter in Tränenflüssigkeiten verwendbar.

Es gibt Methoden zur Gewinnung von Tränenflüssigkeit wie die Abnahme mittels Kapillaren, die bei Spezies mit hoher Tränensekretion angewendet werden können. Dieses Verfahren erfordert Übung, da die Gefahr der Verletzung des Auges durch die Kapillare besteht, besonders wenn die Abnahme mehrere Minuten oder länger dauert. Dies ist besonders dann der Fall, wenn geringe Volumina zur Verfügung stehen, wie im Verlauf einiger Krankheiten ("dry eye syndrome") oder bei Spezies, wie dem Kaninchen, die natürlicherweise eine sehr geringe Tränensekretion haben.

Das Verfahren zur Gewinnung von Tränenflüssigkeit mittels "Schirmer"-Streifen, basierend auf dem Aufsaugen in ein Filter oder Fließpapier, ist ebenfalls nicht optimal. Der Filterstreifen kommt dabei direkt mit der Cornea- oder Conjunktiva-Oberfläche für mehrere Minuten in Berührung, so daß die Gefahr der Verletzung dieser Gewebe besteht. Zudem können Zellen abgelöst werden, die am Filterstreifen adhärierend Meßergebnisse verfälschen können.

Zusätzlich ist die Quantifizierung von Parametern dadurch erschwert, daß eine definierte Volumenaufnahme kaum möglich ist, da der Filterstreifen großflächig ist. Die Bestimmung des aufgesaugten Volumens ist zwar möglich, erfordert jedoch eine Differenzwägung. Diese Schritte haben einen Zeitverlust zur Folge und bieten zusätzliche Fehlermöglichkeiten bei der anschließenden Bestimmung eines Testparameters.

Der vorliegenden Erfindung liegt daher die Aufgabe zugrunde, die Gewinnung von Tränenflüssigkeit in der Weise zu verbessern, daß sie einerseits schnell und für den Probanden schonend vorgenommen werden kann und andererseits die Bestimmung von Komponenten in der gewonnenen Tränenflüssigkeit qualitativ und quantitativ mit hoher Reproduzierbarkeit erlaubt, in dem sie den Einsatz eines definierten Volumens an Tränenflüssigkeit in den Test ermöglicht. Insbesondere sollte die Gewinnung von Tränenflüssigkeit bei solchen Tierspezies, z. B. dem Kaninchen, verbessert werden die natürlicherweise eine sehr geringe Tränensekretion haben.

Die Aufgabe wird durch Bereitstellung eines geeigneten Probenabnahmebestecks sowie eines besonderen Verfahrens der Probenabnahme gelöst.

Die Erfindung betrifft somit ein Probenabnahmebesteck zur Gewinnung eines definierten Volumens Tränenflüssigkeit enthaltend eine in geeigneter Weise abgefüllte für Augen verträgliche Flüssigkeit und ein geformtes saugfähiges Material zur Probennahme.

Bevorzugterweise besteht das geformte saugfähige Material aus einem nicht saugfähigen Trägermaterial und einer darauf fixierten saugfähigen Schicht, die der Probenaufnahme dient.

In einer besonders bevorzugten Ausführungsform enthält das Probenabnahmebesteck einen flexiblen Kunststoffstreifen mit einer darauf fixierten saugfähigen Schicht, die in ihrer Fläche und Höhe begrenzt ist.

Die Erfindung betrifft weiterhin ein Verfahren zur Gewinnung von Tränenflüssigkeit, dadurch gekennzeichnet, daß ein definiertes Volumen einer für Augen verträglichen Flüssigkeit auf die Augenoberfläche oder in den Bindegewebesack gebracht und anschließend ein definiertes Volumen der auf diese Weise vermehrten Tränenflüssigkeit entnommen wird.

Das in das Auge eingebrachte Volumen und das Filterpapier können auf das benötigte Tränenvolumen abgestimmt werden, das in den späteren Testansatz überführt werden muß, um die Komponente nachweisen zu können. Eingebracht werden sollten Volumina zwischen 1 und 500 µl (je nach Größe des Auges von Mensch oder anderen Spezies), beim Menschen, Primaten, Kaninchen, Katze oder Hund bevorzugt zwischen 2 und 200 µl, besonders bevorzugt zwischen 3 und 100 µl und ganz besonders bevorzugt zwischen 5 und 30 µl.

Bestimmte Volumina können durch Aufsaugen mit einem saugfähigen Material gewonnen werden, das in seiner Dimension an das getropfte Volumen angepaßt ist und die rasche Abnahme ermöglicht. Dabei ist das saugfähige Material stets so zu dimensionieren, daß dem maximal aufsaugbaren Volumen ein Überschuß an vermehrter Tränenflüssigkeit gegenübersteht, da nur so gewährleistet werden kann, daß ein reproduzierbares Volumen an Tränenflüssigkeit entnommen wird.

Vorteilhafterweise wird die Dimensionierung des saugfähigen Materials auch an das natürlicherweise in einer bestimmten Spezies zu erwartende Tränenvolumen angepaßt.

In der Praxis wird man mittels der oben beschriebenen Materialien bevorzugt folgendermaßen verfahren:
Ein definiertes Volumen einer für Augen verträglichen Flüssigkeit wird auf die Augenoberfläche oder in den Bindegewebesack getropft. Nach mehrmaligem Öffnen und Schließen des Auges, bevorzugt zwei- bis viermal, zur Durchmischung, wird ein Filterpapier, bevorzugt fixiert an einem Trägerstreifen, auf die Innenseite des unteren Augenlids gelegt, wobei der Trägerstreifen dem Lid zugekehrt ist.

Das Auge kann während der Abnahme geöffnet oder bevorzugt geschlossen bleiben. Nach 2 bis 300 Sekunden, bevorzugt 30 Sekunden, wird der Filterstreifen entfernt und kann für die Bestimmung von Komponenten verwendet werden.

Möglich ist auch die Gewinnung der Flüssigkeit aus dem Filter durch Zentrifugation beispielsweise durch Auflegen auf eine 0,2 µm Membran ("Centricon^{R}"-Röhrchen) zur Entfernung zellulärer Bestandteile und anschließende Analyse, z. B. durch Chromatographie, Elektrophorese, ELISA oder RIA. Auch die Entwicklung oder Freisetzung von Reagenzien oder Farbstoffen am oder im Filter und die Quantifizierung z. B. über Reflexionsphotometrie kann Verwendung finden. Da die Tränenflüssigkeit auch die Beschaffenheit des Blutes wiederspiegeln kann - diese korrespondieren über ein Filtersystem miteinander- z. B. den Glukosespiegel, kann das beschriebene Verfahren auch zur Quantifizierung von löslichen Blutbestandteilen angewendet werden.

Die Quantifizierung von Komponenten, bevorzugt die Bestimmung von Enzymaktivitäten oder -konzentrationen, besonders bevorzugt von Proteaseaktivitäten oder -konzentrationen (z. B. Urokinase, t-PA, Plasmin, Kollagenasen, Cathepsine, Thrombin oder Kallikrein), kann sich an die Gewinnung der Tränenflüssigkeit anschließen.

Das erfindungsgemäße Probenabnahmebesteck eignet sich zur Gewinnung von Tränenflüssigkeit sowohl von Menschen als auch von anderen Spezies mit anschliessender qualitativer und quantitativer Bestimmung von Meßparametern.

Am Beispiel der Konzentrations- und Aktivitätsbestimmung der Protease Plasmin soll das Probennahmebesteck und sein Einsatz beschrieben werden:

### Beispiel 1:

### Materialien:

### Filterpapier (sterilisiert durch Autoklavieren):

MN 818 (®Macherey-Nagel; Düren / Deutschland
6 x 5 mm), fixiert auf einem Plastikstreifen (6 x 90 mm), wobei das Filterpapier 1 mm über die Kante des Plastikstreifens hinausragt.

### Tropflösung

sterile isotonische NaCl-Lösung

### Puffer

40 mM HEPES, 280 mM NaCl, 8 mM KCl, 2 mM MgCl₂, 0,5 g/l Polyoxyethylenglycoldodecylether, pH 7,5

### Enzym-Substrat

H-D-Val-Leu-Lys-pNAx2HCl
S-2251 (Fa. Kabi)

### Standard-Plasmin

Sub-Standard BW lot: 929891, kalibriert an 2nd IRP 77 / 588 NIBSC

### Sammeln der Tränenflüssigkeit:

- Tropfen von 20 µl steriler isotonischer NaCl-Lösung auf die Augenoberfläche oder in den (abgezogenen) Bindegewebesack (unteres Lid); 3maliges Blinzeln mit den Lidern (Durchmischung);
- Auflegen des Teststreifens auf das untere Lid, wobei das Plastikmaterial unten positioniert ist (auf dem Lid aufliegend). Die Augenoberfläche (Cornea / Conjunktiva) sollte nicht berührt werden;
- Das Auge wird für 30 Sek. geschlossen;
- Der vollgesaugte Filterstreifen wird entfernt und die am Plastik adhärente Flüssigkeit mittels eines Fließpapieres abgestreift;
- Der Filterstreifen wird in eine vorbereitete Küvette plaziert, so daß der Streifen nicht im "Strahlengang" der Küvette liegt. Das Filterpapier ist dem Lumen der Küvette zugewandt. Die Küvette wird bei 37°C inkubiert.

### Bestimmung der Plasminaktivität:

Die Plasminaktivität wird photometrisch durch Spaltung des chromogenen Substrates S-2251 und Freisetzung von Para-Nitroanilid bei 405 nm bestimmt. Anhand einer mit Standard-Plasmin erstellten Eichkurve wird die Plasminaktivität quantifiziert.

Die Standardkurve wird erstellt, indem Filterstreifen für ca. 10 Sek. an die vorbereitete Standardlösung angelegt werden (ca. 1 mm eintauchen), die überschüssige Flüssigkeit an Plastik abgestreift und der Streifen in die Küvette eingeführt wird.

Die Küvetten werden vorbereitet durch Pipettieren von jel150 µl Aqua dest. und 240 µl HEPES-Puffer.

Nach Zugabe von 100 µl Substrat-Lösung (4mM) wird die OD405nm nach unterschiedlichen Inkubationszeiten gemessen. Der über den Küvettenrand ragende Plastikanteil des Trägers kann zur besseren Plazierung im Photometer abgetrennt werden.

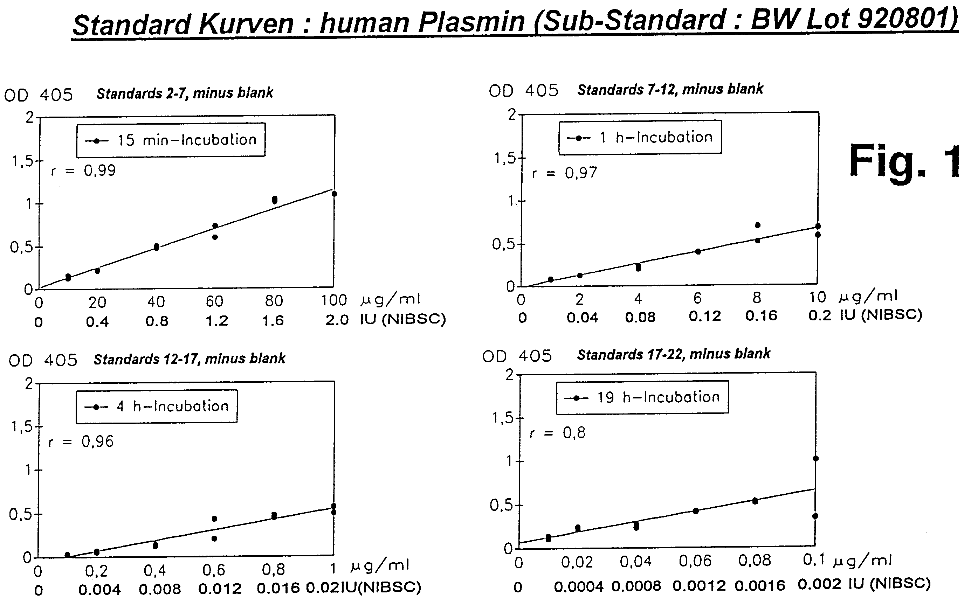

Zur Abgreifung eines möglichst weiten Meßbereiches kann so verfahren werden, daß die einmal angesetzte Standardkurve samt Proben zu mehreren Zeitpunkten gemessen wird. In diesem Beispiel ist die Bestimmung von Plasminaktivität in einem Meßbereich von 0,05 bis 100 µg/ml durchführbar. Gemessen nach - bei Anwendung der angegebenen Bedingungen - 15, 60 und 240 Min. (siehe Fig. 1).

Die Plasminaktivität und Konzentration wird anhand der Standardkurve (in einem gut auswertbaren, linearen Meßbereich) quantifiziert. Es empfiehlt sich, unterschiedliche Meßbereiche nach verschiedenen Inkubationszeiten zu verwenden:

| | | |
|---|---|---|
| 15 Min. Inkubation | 2,0 IU/ml | - 0,2 IU/ml |
| | 100, 0 µg/ml | -10,0 µg/ml |
| 60 Min. Inkubation | 0,2 IU/ml | - 0,02 IU/ml |
| | 10,0 µg/ml | -1,0 µg/ml |
| 240 Min. Inkubation | 0,02 IU/ml | - 0,002 IU/ml |
| | 1,0 µg/ml | -0,1 µg/ml |

Inkubation für >12 Stunden erlaubt die Quantifizierung von Plasminspiegeln auch <0,002 IU/ml (<0,1 µg/ml).

### Ergebnis:

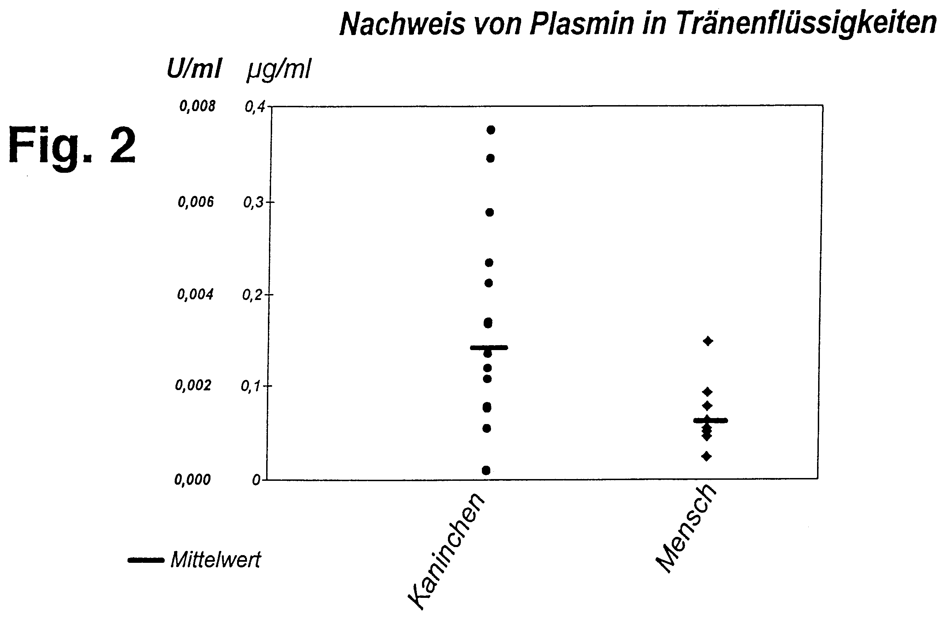

- Standard-Kurven (Fig. 1)
- "Normalwerte" für Menschen und Kaninchen wurden wie in Fig. 2 dargestellt bestimmt.

Diese Normalwerte sind trotz der Streuung der Einzelwerte zur Abgrenzung pathologischer Zustände brauchbar, da die pathologischen Werte um ein Mehrfaches größer sind.

In ähnlicher Weise kann z. B. mit weiteren Proteasen und anderen Enzymen verfahren werden.

## Patentansprüche

1. Probenabnahmebesteck zur Gewinnung eines definierten Volumens Tränenflüssigkeit, dadurch gekennzeichnet, daß es eine in geeigneter Weise abgefüllte, für Augen verträgliche Flüssigkeit und ein geformtes saugfähiges Material zur Probennahme enthält.

2. Probenabnahmebesteck gemäß Anspruch 1, dadurch gekennzeichnet, daß das geformte saugfähige Material zur Probennahme aus einem nicht-saugfähigen Trägermaterial und einer darauf fixierten saugfähigen Schicht besteht.

3. Probenabnahmebesteck gemäß Anspruch 2, dadurch gekennzeichnet, daß das Trägermaterial ein flexibler Kunststoffstreifen ist und die darauf fixierte saugfähige Schicht in ihrer Fläche und Höhe begrenzt ist.

4. Verfahren zur Gewinnung von Tränenflüssigkeit, dadurch gekennzeichnet, daß
a) ein definiertes Volumen einer für Augen verträglichen Flüssigkeit auf die Augenoberfläche oder in den Bindegewebesack gebracht und
b) ein definiertes Volumen der vermehrten Tränenflüssigkeit entnommen wird.

5. Verfahren nach Anspruch 4, dadurch gekennzeichnet, daß zwischen 1 und 500 µl eingebracht werden.

6. Verwendung von gemäß Anspruch 4 gewonnener Tränenflüssigkeit zur Bestimmung von Komponenten in der Tränenflüssigkeit.

7. Verwendung von gemäß Anspruch 6 bestimmten Komponentenwerten zur Diagnose.
